# EUROPEAN PATENT APPLICATION

(11) **EP 4 140 510 A1**
(43) Date of publication of application: **01.03.2023**
(21) Application number: 21793567.5
(22) Date of filing: 20.04.2021
(51) Int. Cl.: A61L 27/04, A61L 27/12, A61L 27/36, A61L 27/40, A61L 27/50, A61L 27/54, A61F 2/34

(54) **SHELL FOR ARTIFICIAL JOINT AND METHOD FOR PRODUCING SAME**

(30) Priority: 22.04.2020 JP 2020076203
(71) Applicant: Kyocera Corporation, Kyoto-shi Kyoto 612-8501 (JP)
(72) Inventor: NODA, Iwao, Kyoto-shi, Kyoto 612-8501 (JP)
(74) Representative: TBK
(86) International application number: PCT/JP2021/016059
(87) International publication number: WO 2021/215441

(57) **Abstract**

In the present disclosure, an artificial joint shell includes a base and a coating film. The base has a cup shape and includes an outer surface including a first region and a second region adjacent to the first region. The coating film contains a calcium phosphate-based material and an antimicrobial material and is disposed across the first region and the second region of the outer surface of the base. A surface of the coating film in the first region has a larger surface roughness than a surface of the coating film in the second region.

## Description

### TECHNICAL FIELD

The present disclosure relates to an artificial joint shell and a method for manufacturing the artificial joint shell.

### BACKGROUND OF INVENTION

The use of biological implants for the treatment of both bone injuries and diseases is constantly expanding with an increase in active population and aging population. In such a situation, a known biological implant is provided with a coating from the viewpoint of antimicrobial properties, fixation properties to a bone, and the like.

For example, Patent Document 1 describes a coating for a medical implant, wherein a part of the coating contains a bone-binding agent and an antimicrobial metal agent containing silver.

### CITATION LIST

### PATENT LITERATURE

Patent Document 1: JP 2011-512959 T

### SUMMARY

In the present disclosure, an artificial joint shell includes a base and a coating film. The base has a cup shape and includes an outer surface including a first region and a second region adjacent to the first region. The coating film contains a calcium phosphate-based material and an antimicrobial material and is disposed across the first region and the second region of the outer surface of the base. A surface of the coating film in the first region has a larger surface roughness than a surface of the coating film in the second region.

In the present disclosure, a method for manufacturing an artificial joint shell includes: preparing a base having a cup shape, disposing a first protective material such that a first part of an outer surface of the base is exposed and a second part of the outer surface is protected, first surface roughening of forming a first rough surface portion at the first part exposed from the first protective material, removing the first protective material, and coating film forming of forming a coating film at the first rough surface portion and at least a part of the second part of the outer surface, the coating film containing a calcium phosphate-based material and an antimicrobial material.

### BRIEF DESCRIPTION OF THE DRAWINGS

FIG. 1 is a schematic view illustrating an artificial joint shell according to an embodiment when viewed from above.
FIG. 2 is a schematic view illustrating an artificial joint shell according to an embodiment when viewed obliquely from below.
FIG. 3 is a schematic view illustrating a cross-section of an artificial joint shell according to an embodiment taken along a line A-A' in FIG. 1.
FIG. 4 is a schematic view illustrating an example of a cross-section of an artificial joint shell according to an embodiment when a view of a region X in FIG. 3 is enlarged.
FIG. 5 is a schematic view illustrating an example of a cross-section of an artificial joint shell according to an embodiment when a view of the region X in FIG. 3 is enlarged.
FIG. 6 is a schematic view illustrating an example of a cross-section of an artificial joint shell according to an embodiment when a view of the region X in FIG. 3 is enlarged.
FIG. 7 is a schematic view illustrating an example of a cross-section of an artificial joint shell according to an embodiment when a view of the region X in FIG. 3 is enlarged.
FIG. 8 is a schematic view illustrating an example of a cross-section of an artificial joint shell according to an embodiment taken along a line B-B' in FIG. 1.
FIG. 9 is a schematic view illustrating an example of a cross-section of an artificial joint shell according to an embodiment when a view of a region Y in FIG. 8 is enlarged.
FIG. 10 is a schematic view illustrating an artificial hip joint according to an embodiment.
FIG. 11 is a schematic view illustrating an example in which an instrument is connected to an artificial hip joint shell according to an embodiment.
FIG. 12 is a schematic view illustrating an example in which an instrument is connected to an artificial hip joint shell according to an embodiment.
FIG. 13 is a flowchart illustrating a method for manufacturing an artificial joint shell according to an embodiment.

### DESCRIPTION OF EMBODIMENTS

Hereinafter, one embodiment will be described in detail. Note that, unless otherwise specified in the present specification, "A to B", which represents a numerical range, means "A or more and B or less".

### 1. Artificial Joint Shell

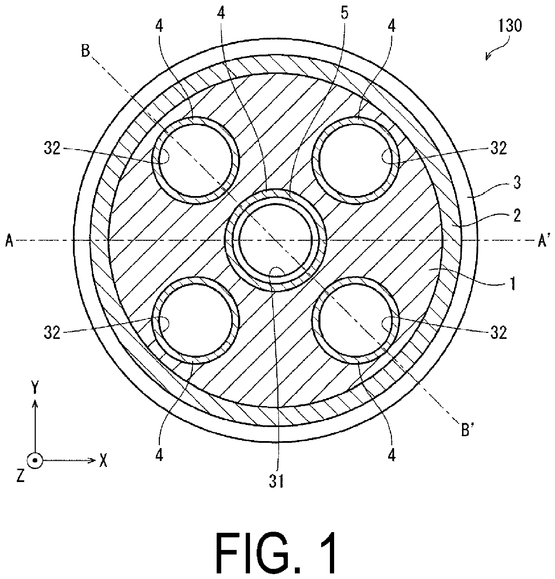

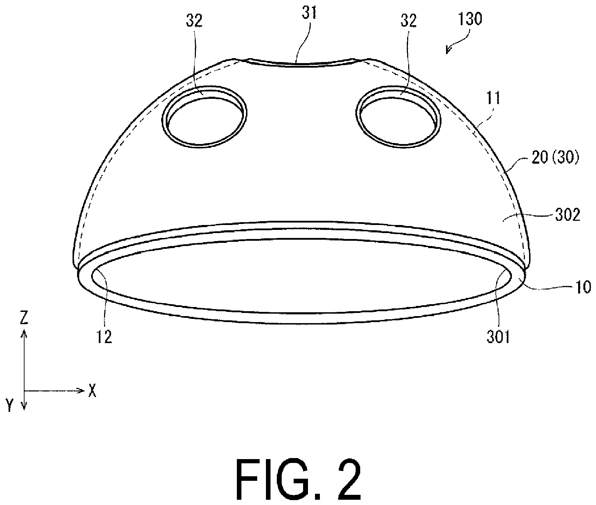

First, a configuration of an artificial joint shell 130 according to an embodiment will be described with reference to FIGs. 1 to 4. FIG. 2 is a perspective view of the artificial joint shell 130. As illustrated in FIG. 2, the artificial joint shell 130 has a hemispherical shape, for example, like one obtained by dividing a spherical member into halves, and a coating film 20 is disposed on the outer surface of a base 10 having a hemispherical shape. The coating film 20 contains a calcium phosphate-based material and an antimicrobial material. Note that the calcium phosphate-based material has an effect of improving fixation properties to a bone. Also, the antimicrobial material has an effect of reducing adhesion and growth of bacteria.

The base 10 is a member at which a liner 120 described below is disposed. The base 10 has a cup shape including a recessed portion, and the recessed portion includes an opening portion 12 opened in one direction in the base 10. Accordingly, the liner 120 is disposed in the base 10 via the opening portion 12. In the present embodiment, the base 10 includes a layered member 30 between the outer surface and the coating film 20. The base 10 further includes a screw hole 31 and a counterbored hole 32. In the present invention, the base 10 need not include the layered member 30, the screw hole 31, and the counterbored hole 32.

As illustrated in FIG. 2, in the present embodiment, a plane of the artificial joint shell 130 corresponding to a cross-section obtained by dividing a sphere so as to include the center of the sphere is defined as an XY plane, and a direction perpendicular to the XY plane and directed toward the vertex of a hemisphere is defined as a Z direction. The Z direction is referred to as the upper side of the artificial joint shell 130.

FIG. 1 is a schematic view illustrating the artificial joint shell 130 when viewed from above. As illustrated in FIG. 1, the artificial joint shell 130 has a hemispherical shape as described above. The base 10 has a hemispherical shape, and the outer surface of the base 10 includes a first surface 301 (lower surface, see FIG. 2) facing downward (a negative direction of the Z axis) of the base 10, and a second surface 302 (outer peripheral surface, see FIG. 2) different from the first surface 301. The first surface 301 is a surface defining the opening portion 12, and the second surface 302 is a surface to be in contact with a pelvis during surgery. In the present embodiment, the first surface 301 is a flat surface, and the second surface 302 is a convex curved surface.

Note that the cup shape of the base 10 may be, for example, a spherical segment shape obtained by cutting out a region smaller than a half of a sphere from the sphere or a shape of the remainder of the sphere after the spherical segment is cut out, in addition to the hemispherical shape formed by dividing the spherical member into halves. Also, for example, the cup shape may be a semi-ellipsoid shape formed by dividing an ellipsoid so as to include the center of the ellipsoid, an ellipsoid segment shape obtained by cutting out a region smaller than a half of the ellipsoid from the ellipsoid, or a shape of the remainder of the ellipsoid after the ellipsoid segment shape is cut out.

The convex curved surface of the second surface 302 is only required to be convex when viewed as a whole, and may be locally concave. Also, the convex curved surface of the second surface 302 is only required to be a curved surface when viewed as a whole, and may be locally flat. An inner surface of the recessed portion of the base 10 includes a concave curved surface. The inner surface is only required to be concave when viewed as a whole, and may be locally convex. Also, the concave curved surface of the inner surface is only required to be a curved surface when viewed as a whole, and may be locally flat.

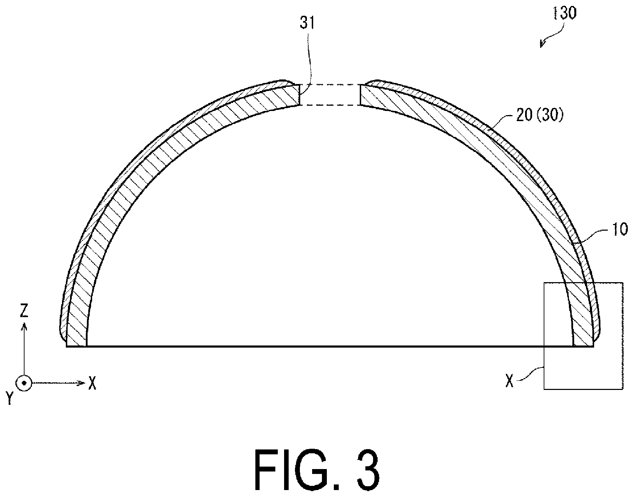

The opening portion 12 may be a straight line when the base 10 is viewed from the side as illustrated in FIG. 3, but may be an upward convex curve or a downward convex curve, or may have a wave shape including two or more upward convex portions and/or downward convex portions.

In the present embodiment, the outer surface of the base 10 includes a first region 1 and a second region 2, and includes a third region 3. Note that, in the present invention, the base 10 need not include the third region 3. Also, in the present embodiment, the base 10 is provided with the screw hole 31 and the counterbored hole 32 described above. In this case, the base 10 may further include a fourth region 4 and a fifth region 5. Note that, in the present invention, the base 10 need not include the fourth region 4 and the fifth region 5.

The first region 1 is a region that is disposed in at least part of a region of the outer surface of the base 10 excluding the end portion of the second surface 302. In the case where the screw hole 31 and the counterbored hole 32 are disposed in the base 10, the first region 1 is a region that is disposed in at least part of a region of the outer surface of the base 10 excluding the end portion of the second surface 302, and the peripheries of the screw hole 31 and the counterbored hole 32.

The second region 2 is a region that is adjacent to the first region 1 and is disposed between the first region 1 and the opening portion 12 of the base 10. The third region 3 is a region that is adjacent to the second region 2 and is disposed between the second region 2 and the opening portion 12. The fourth region 4 is a region that is adjacent to the first region 1 and is disposed between the first region 1 and the screw hole 31 or the counterbored hole 32. The fifth region 5 is a region that is adjacent to the fourth region 4 and is disposed between the fourth region 4 and the screw hole 31.

The coating film 20 is disposed across the first region 1 and the second region 2 of the outer surface of the base 10, and the surface of the coating film in the first region 1 has a larger surface roughness than the surface of the coating film in the second region 2.

The coating film 20 located in the second region 2 may include an end portion 21 and a base portion 22 located closer to the first region 1 than the end portion 21 is to the first region 1, and the thickness of the base portion 22 may be larger than the thickness of end portion 21.

In the case where the third region 3 is disposed at the base 10, the outer surface of the base 10 located in the third region 3 is exposed from the coating film 20. The surface of the coating film 20 in the second region 2 may have a larger surface roughness than the outer surface in the third region 3.

In the case where the fourth region 4 is disposed at the base 10, the outer surface located in the fourth region 4 has a smaller surface roughness than the surface of the coating film in the first region 1. The coating film 20 is disposed at at least part of the outer surface in the fourth region 4. In the case where the fifth region 5 is disposed at the base 10, the entire outer surface in the fifth region 5 is exposed from the coating film 20.

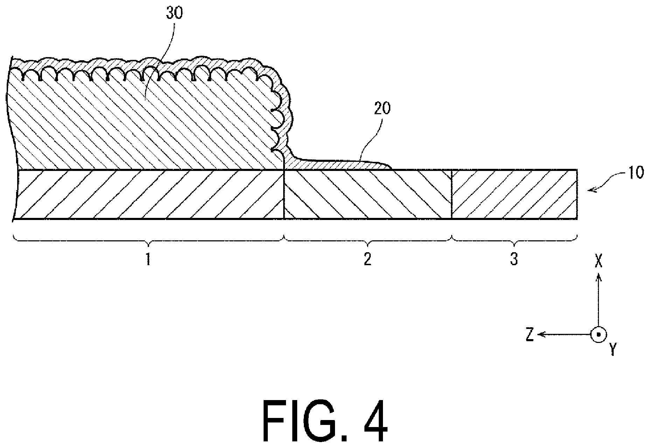

FIG. 3 is a view illustrating a cross-section of the artificial joint shell 130 taken along a line A-A' in FIG. 1. FIGs. 4 to 7 are schematic views illustrating enlarged views of the region X in FIG. 3.

At the base 10, the first region 1 and the second region 2 are located in sequence. In the case where the base 10 includes the third region 3, the first region 1, the second region 2, and the third region 3 are located in sequence as illustrated in FIG. 4. The first region 1 and the second region 2, or the first region 1, the second region 2, and the third region 3 can be understood as being located in sequence in a direction parallel to the surface of the base 10. In the case where the base 10 does not include the third region, the second region 2 corresponds to the end portion proximate to the opening portion 12 of the base 10, and in the case where the base 10 includes the third region, the third region 3 corresponds to the end portion proximate to the opening portion 12 of the base 10.

Note that, in FIGs. 4 to 7, the first region 1, the second region 2, and the third region 3 are hatched differently for descriptive purposes, but these regions need not be different components. The base 10 may be composed of one component. The first region 1, the second region 2, and the third region 3 can be distinguished in terms of design by, for example, the presence or absence of the coating film or the presence or absence of a rough surface to be described later. The coating film 20 is located across the first region 1 and the second region 2. The third region 3 is exposed from the coating film 20. In the case where the base 10 includes the third region 3, the surface of the coating film 20 located in the first region 1 has a larger surface roughness than the surface of the base 10 in the third region 3.

Examples of surface roughness indices include arithmetic mean roughness Sa (ISO 25178). The surface roughness (Sa) of the coating film 20 located in the first region 1 may be set to, for example, 10 to 80 µm, or may be set to 20 to 80 µm, or may be set to 30 to 70 µm. The surface roughness (Sa) of the base 10 in the third region 3 is only required to be set to less than 1.0 µm, for example.

Note that the surface roughness Sa of the first region 1 can be determined based on the measurement result of the entire first region 1. Similarly, the surface roughness Sa of the second region 2 can be determined based on the measurement result of the entire second region 2, and the surface roughness Sa of the third region 3 can be determined based on the measurement result of the entire third region 3. For example, the first region 1 may include a portion in which the surface roughness is locally smaller than the surface roughness of the base 10 in the second region 2, and the second region 2 may include a portion in which the surface roughness is locally larger than the surface roughness of the coating film 20 in the first region 1.

The surface roughness of the coating film 20 or the surface roughness of the base 10 is only required to be measured, for example, by a stylus method or an optical method. Also, the surface roughness is only required to be measured in accordance with "ISO 25178", for example. Note that methods for measuring surface roughness are not limited to the above-described methods.

Here, there has been room for improvement in an artificial joint shell in terms of achieving the antimicrobial properties and the control of fixation properties to a bone in a compatible manner. In other words, for example, in a case where the surface of the artificial joint shell includes a region having a large surface roughness, there is a possibility that an end portion of the region having a large surface roughness may cause irritation to a bone or soft tissue and may become a start point of microbial infection.

In the artificial joint shell 130 according to the present disclosure, the surface roughness of the coating film 20 in the first region 1 is larger than the surface roughness of the second region 2. Thus, the fixation properties to a bone can be sufficiently achieved. The coating film 20 extending across the first region 1 and the second region 2 is further included. Accordingly, an end portion of the first region can be covered with the coating film 20 having antimicrobial properties, and thus the artificial joint shell 130 can achieve the fixation properties to a bone and the antimicrobial properties in a compatible manner.

The base 10 can be made of metal, ceramic, or plastic. Examples of the metal include stainless steel alloys, cobalt chromium alloys, titanium, and titanium alloys. As the titanium alloys, alloys added with at least one selected from the group consisting of aluminum, tin, zirconium, molybdenum, nickel, palladium, tantalum, niobium, vanadium, platinum, and the like can be used. Examples of the ceramic include alumina, zirconia, and alumina-zirconia composite ceramic. Examples of the plastic include polyethylene, fluorine-based resin, epoxy resin, polyetheretherketone (PEEK) resin, and Bakelite. Note that in the present embodiment, the base 10 is made of titanium alloy.

At least part of the first region 1 is covered with the coating film 20. That is, the entire surface of the first region 1 may be covered with the coating film 20, or only part of the first region 1 may be covered with the coating film 20. The surface of the coating film 20 located in the first region 1 has a larger surface roughness than the surface of the coating film 20 in the second region 2. Providing a region having a large surface roughness in this manner can improve the fixation properties to a bone. For example, the surface roughness of the coating film 20 located in the first region 1 can be increased by forming a rough surface in the first region 1 and covering the rough surface with the coating film 20.

Note that the surface roughness of the rough surface formed in the first region 1 is set to be larger than the surface roughness of the second region 2. Thus, the surface roughness of the coating film 20 located in the first region 1 can be made larger than the surface roughness of the coating film 20 located in the second region 2. The surface roughness of the rough surface formed in the first region 1 may be set to 10 to 80 µm, or may be set to 20 to 80 µm, or may be set to 30 to 70 µm, for example. Note that the surface roughness of the rough surface can be measured, for example, by cutting the artificial joint shell 130 and observing the cut surface by an SEM or the like. Note that, in the present invention, the artificial joint shell 130 is not limited to a configuration in which the surface roughness of the first region 1 is larger than the surface roughness of the second region 2.

The artificial joint shell 130 may further include the layered member 30. The layered member 30 may be disposed on the first region 1. Accordingly, as illustrated in FIG. 4, the first region 1 is higher than a region where the layered member 30 is not provided (for example, the second region 2). Thus, when the artificial joint shell 130 is embedded in a bone, the first region 1 can primarily contact the bone. In the present specification, the "layered member" means a member that is stacked on the base 10 and is different from the coating film 20. For example, the surface of the layered member 30 may be rough. In this way, the region primarily in contact with a bone can have a rough surface. The layered member 30 may be formed by a thermal spraying method as described below. Alternatively, the layered member 30 may be formed to have a porous structure. In the present embodiment, the surface roughness of the layered member 3 is set to be larger than the surface roughness of the second region 2.

Note that the lower limit of the height of the layered member 30 is only required to be set to, for example, 100 µm or more, and may be set to 300 µm or more. The upper limit is only required to be set to, for example, 1000 µm or less, and may be set to 700 µm or less. The surface roughness of the layered member 30 may be set to, for example, 10 to 80 µm, or may be set to 20 to 80 µm, or may be set to 30 to 70 µm.

Note that the base 10 may be formed into a shape that enables the first region 1 to primarily contact a bone without the layered member 30. For example, the first region 1 may have a shape raised with respect to the second region 2 and the third region 3.

As a material of the layered member 30, the materials that have been exemplified as the material of the base 10 can be used. For example, the layered member 30 may be made of metal. The layered member 30 and the base 10 may be made of the same material, or may be made of different materials. Accordingly, a sufficient strength can be achieved. Note that, in the present embodiment, the layered member 30 is formed of the same material as the base 10. In the present embodiment, the layered member 30 is formed of titanium alloy.

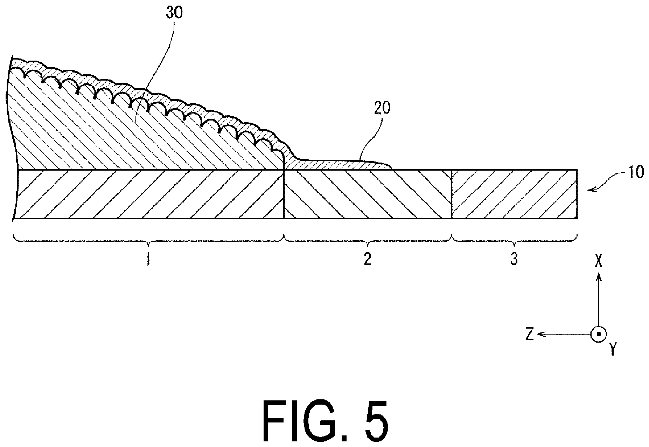

The layered member 30 may include an edge having a lower height than an inner portion of the layered member 30. In this specification, "an inner portion of the layered member" means an inner side of the layered member 30 in a surface direction. FIG. 5 illustrates the layered member 30 including an edge having a lower height than the inner portion. Accordingly, the concentration of stress at the edge of the layered member 30 can be reduced.

The coating film 20 contains a calcium phosphate-based material and an antimicrobial material. Examples of the calcium phosphate-based material include one type or two types or more of mixtures selected from the group consisting of hydroxyapatite, α-tertiary calcium phosphate, β-tertiary calcium phosphate, quaternary calcium phosphate, octacalcium phosphate, and calcium phosphate-based glass. As the antimicrobial material, a natural antimicrobial agent, an organic antimicrobial agent, or an inorganic antimicrobial agent can be used. For example, hinokitiol can be used as a natural antimicrobial agent, benzalkonium chloride can be used as an organic antimicrobial agent, and a metal can be used as an inorganic antimicrobial agent. Examples of the metal include silver, copper, and zinc. In addition to the calcium phosphate-based material and the antimicrobial material, the coating film 20 may contain a glass ceramic, and may further contain an antimicrobial agent such as penicillin and vancomycin.

The concentration of the antimicrobial material in the coating film 20 may be, for example, from 0.05 wt% to 3.00 wt%, from 0.05 wt% to 2.50 wt%, from 0.05 wt% to 1.00 wt%, or from 0.1 wt% to 1.00 wt%. When the concentration of the antimicrobial material is 0.05 wt% or more, sufficient antimicrobial properties can be achieved. Further, when the concentration of the antimicrobial material is 3.00 wt% or less, the impact on living tissue can be reduced.

The coating film 20 may be disposed on the layered member 30. As described above, the layered member 30 can primarily come in contact with a bone. The coating film 20 is disposed on the layered member 30, which can further improve the fixation properties to a bone and the antimicrobial properties.

The height of the layered member 30 may be greater than the thickness of the coating film 20. Accordingly, the region in which the layered member 30 is formed becomes higher than the region in which only the coating film 20 is formed, and thus the region in which the layered member 30 is formed can primarily contact a bone. The thickness of the coating film 20 is only required to be set to, for example, less than 100 µm, and may be set to less than 50 µm. In addition, the thickness of the coating film 20 is only required to be set to, for example, 5 µm or more.

At least part of the second region 2 is covered with the coating film 20. That is, the entire surface of the second region 2 may be covered with the coating film 20, or only part thereof may be covered with the coating film 20. In the present specification, the coating film 20 is "located across the first region and the second region", which means that at least part of the boundary between the first region 1 and the second region 2 is covered with the coating film 20. That is, the entire boundary between the first region 1 and the second region 2 may be covered with the coating film 20, or only part thereof may be covered with the coating film 20. When the layered member 30 is disposed in the first region 1, the coating film 20 may extend from the first region 1 to the second region 2 so as to cover the edge of the layered member 30. That is, the edge of the layered member 30 can be covered so as not to be exposed from the coating film 20. Accordingly, bacterial growth can be further reduced.

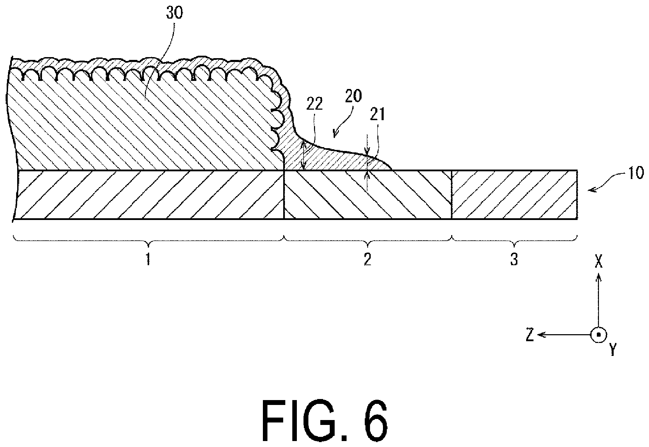

The coating film 20 located in the second region 2 may include an end portion 21 and a base portion 22 located closer to the first region 1 than the end portion 21 is to the first region 1. In the present specification, "the end portion of the coating film located in the second region" refers to a region of the coating film 20 located in the second region 2, the region being located closer to the boundary between the second region 2 and the third region 3. Also, "the base portion of the coating film located in the second region" refers to a region of the coating film 20 located in the second region 2, the region being located closer to the boundary between the first region 1 and the second region 2. Here, the thickness of the base portion 22 may be larger than the thickness of the end portion 21. FIG. 6 illustrates the coating film 20 in which the thickness of the base portion 22 is larger than the thickness of the end portion 21. Accordingly, the concentration of stress at and in the vicinity of the boundary between the first region 1 and the second region 2 can be reduced, and thus the peeling of the coating film 20 can be reduced. In the case where the layered member 30 is disposed in the first region 1, the concentration of stress at and in the vicinity of the edge of the layered member 30 can be reduced.

The third region 3 is exposed from the coating film 20. A region where the coating film 20 is disposed and a region exposed from the coating film 20 can be distinguished from each other by an elemental analysis of the surface of each region. A method for the elemental analysis can be performed, for example, by mapping surface elements using an energy dispersive X-ray (EDX) analyzer that is an auxiliary device of a typical scanning electron microscope (SEM). Alternatively, surface analysis methods such as X-ray photoelectron spectroscopy, Auger electron spectroscopy, and secondary ion mass spectrometry may be used. Still alternatively, a sample obtained by mechanically scraping off the surface of each region may be chemically analyzed for detection of elements. For example, phosphorus, calcium, antimicrobial components, and the like are detected from the surface of at least part of the first region 1 and the surface of at least part of the second region 2 where the coating film 20 is disposed. From the surface of the third region 3, elements constituting the base 10 are detected, and phosphorus, calcium, antimicrobial components, and the like are not detected, or are detected at a noise level or lower.

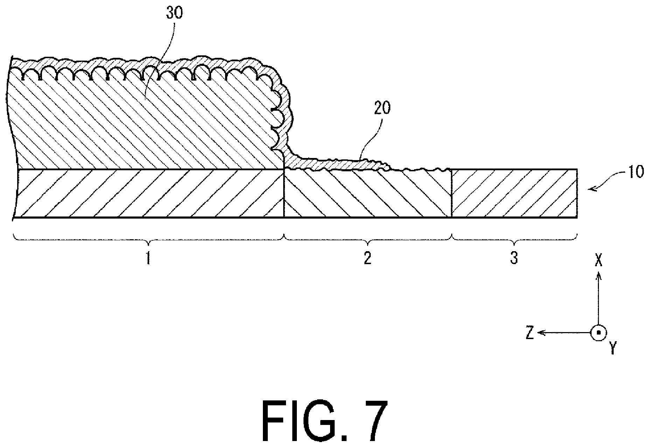

The surface of the coating film 20 located in the second region 2 may have a smaller surface roughness than the surface of the coating film 20 located in the first region 1. FIG. 7 illustrates an aspect in which the surface roughness of the coating film 20 located in the second region 2 is smaller than the surface roughness of the coating film 20 located in the first region 1. Accordingly, the fixation properties to a bone and the antimicrobial properties can be sufficiently achieved in the first region 1. In addition, excessive physical irritation to soft tissue can be reduced in the second region 2.

Note that the surface roughness (Sa) of the coating film 20 located in the first region 1 may be set to, for example, 10 to 80 µm, or may be set to 20 to 80 µm, or may be set to 30 to 70 µm. The surface roughness (Sa) of the coating film 20 located in the second region 2 is only required to be set to, for example, 0.1 to 10 µm.

The surface of the coating film 20 located in the second region 2 may have a larger surface roughness than the outer surface of the base 10 in the third region 3. FIG. 7 illustrates an aspect in which the surface roughness of the coating film 20 located in the second region 2 is larger than the surface roughness of the base 10 in the third region 3. Accordingly, in the second region 2, the adhesion between the coating film 20 and the base 10 can be improved, and thus the fixation properties to a bone and the antimicrobial properties can be sufficiently achieved. In addition, excessive physical irritation to soft tissue can be reduced in the third region 3. Note that the surface roughness (Sa) of the base 10 in the third region 3 is only required to be set to, for example, less than 1 µm.

As illustrated in FIG. 7, the surface roughness of the outer surface of the base 10 in the second region 2 may be larger than the surface roughness of the outer surface of the base 10 in the third region 3. Accordingly, in the second region 2, the adhesion between the coating film 20 and the base 10 can be improved, and thus the peeling of the coating film 20 can be reduced. Note that the surface roughness (Sa) of the base 10 in the second region 2 is only required to be set to 0.1 µm or more and less than 10 µm, and may be set to less than 2.0 µm.

In the present embodiment, the surface roughness of the rough surface of the base 10 in the first region 1 (including the rough surface of the layered member 30) is set to be larger than the surface roughness of the base 10 in the third region 3.

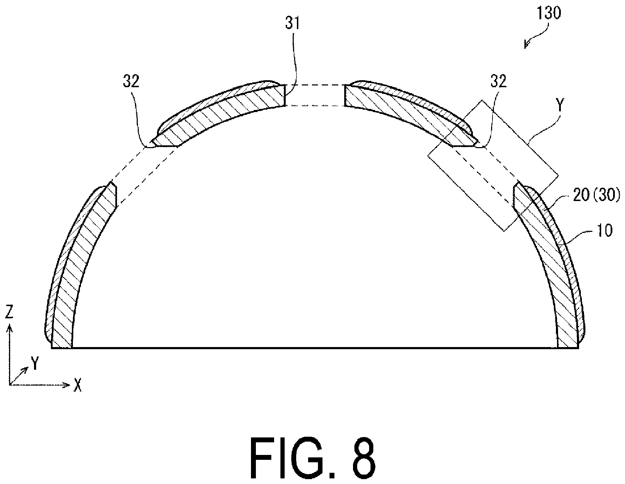

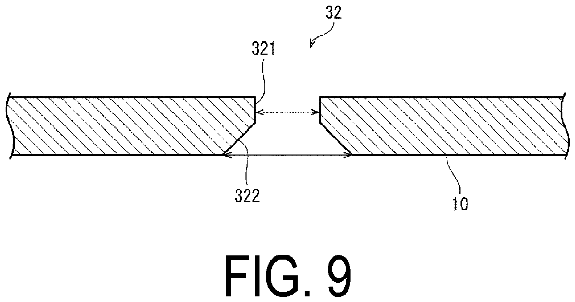

FIG. 8 is a diagram illustrating a cross-section of the artificial joint shell 130 taken along a line B-B' in FIG. 1. FIG. 9 is an enlarged schematic view of the region Y in FIG. 8. As illustrated in FIG. 9, regarding the counterbored hole 32, the width of a penetrating portion 321 (second penetrating portion) proximate to the outer surface is smaller than the width of a penetrating portion 322 (first penetrating portion) proximate to the opening portion 12. That is, the counterbored hole 32 includes the penetrating portion 322 and the penetrating portion 321 that is connected to the penetrating portion 322 and the outer surface and has a smaller width than the penetrating portion 322.

That is, the base 10 may include at least one through hole (the screw hole 31 or the counterbored hole 32) penetrating the outer surface and the inner surface (proximate to the opening portion 12) of the base 10. At least one through hole (the screw hole 31) is disposed at a position further from the opening portion 12 than another position (the counterbored hole 32). It can be said that the counterbored hole 32, which is the other through hole, includes the penetrating portion 322 and the penetrating portion 321 that is connected to the penetrating portion 322 and the outer surface and has a smaller width than the penetrating portion 322.

Note that the artificial joint shell 130 may include one or more fins.

### Screw Hole 31 and Counterbored Hole 32

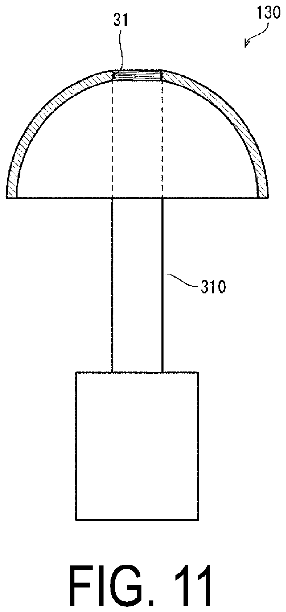

The screw hole 31 is, for example, used to connect an instrument to the artificial joint shell 130 during surgery. For example, as illustrated in FIG. 11, by fixing a distal end portion of a retainer 310 to the screw hole 31 of the artificial joint shell 130, the artificial joint shell 130 can be retained by the retainer 310, and the artificial joint shell 130 can be moved to a desired position.

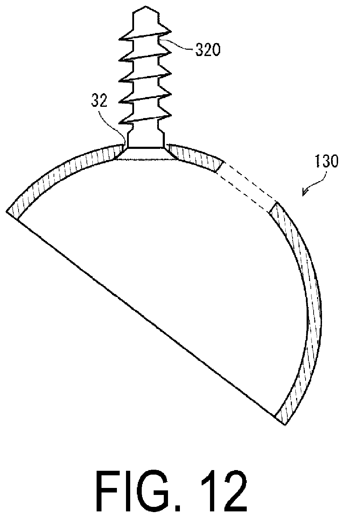

The counterbored hole 32 can be used, for example, to secure the artificial joint shell 130 to a coxal bone 93 with a screw 320 as illustrated in FIG. 12.

With the above-described configuration, the artificial joint shell 130 according to the present disclosure includes the rough surface portion and thus can further improve osteoconductive properties and bone fixation properties. Further, the coating film covers the rough surface portion so as not to expose an end portion of the rough surface portion, and thus can suppress bacterial growth from the end portion of the rough surface portion.

An edge portion of the cup shape, which is likely to be subjected to stress, does not have a rough surface, and thus physical irritation to soft tissue can be reduced. Also, since an edge of the shell is sunken, irritation at the edge can be reduced.

The layered member is made of metal and thus can have sufficient strength. Also, with the coating film formed on the layered member, the adhesion to a bone and the antimicrobial properties are further improved.

Further, since the height of an edge of a metal layer is lower than the height of an inner portion thereof, concentration of stress at an edge of the rough surface portion can be suppressed.

In addition, peeling of an overcoat from an end portion can also be suppressed.

The fixation properties to a bone and the antimicrobial properties are sufficiently achieved in the second region 2. In addition, since the surface roughness of the surface of the third region 3 is small, irritation to soft tissue can be further reduced.

The coating film is provided on the outer surface of the base 10 in the second region having a larger surface roughness than the outer surface of the base 10 in the third region 3, and thus adhesion between the coating film and the base member is improved and the coating film is less likely to be peeled off from the base member.

In addition, fixation to a jig or an acetabulum at a desired position can be performed.

In the case where the screw hole 31 and the counterbored hole 32 are disposed in the base 10, the first region 1, the fourth region 4, and the screw hole 31 or the counterbored hole 32 are located in sequence in the base 10. In the case where the base 10 includes the fifth region 5, the first region 1, the fourth region 4, the fifth region 5, and the screw hole 31 are located in sequence in the base 10.

The first region 1, the fourth region 4, and the screw hole 31 or the counterbored hole 32 described above, or the first region 1, the fourth region 4, the fifth region 5, and the screw hole 31 described above can be understood as being located in sequence in a direction parallel to the surface of the base 10. Thus, in the case where the base 10 does not include the fifth region 5, the fourth region 4 corresponds to end portions proximate to the screw hole 31 and the counterbored hole 32 of the base 10. In the case where the base 10 includes the fifth region 5, the fifth region 5 corresponds to an end portion proximate to the screw hole 31 of the base 10, and the fourth region 4 corresponds to an end portion proximate to the counterbored hole 32 of the base 10.

The entire surface of the fourth region may be covered with the coating film 20, or only a part of the fourth region may be covered with the coating film 20. The boundary between the first region and the fourth region need not be covered with the coating film 20, or may be covered with the coating film 20 in whole or only in part. In the case where the boundary between the first region and the fourth region 4 is covered with the coating film 20 and where the layered member 30 is disposed in the first region 1, the coating film 20 may extend from the first region 1 to the fourth region 4 so as to cover an edge of the layered member 30. That is, the edge of the layered member 30 can be covered so as not to be exposed from the coating film 20. Accordingly, bacterial growth can be further reduced.

The fifth region 5 is exposed from the coating film 20. A region where the coating film 20 is disposed and a region exposed from the coating film 20 can be distinguished from each other by an elemental analysis of the surface of each region. A method for the elemental analysis can be performed, for example, by mapping surface elements using an energy dispersive X-ray (EDX) analyzer that is an auxiliary device of a typical scanning electron microscope (SEM). Alternatively, surface analysis methods such as X-ray photoelectron spectroscopy, Auger electron spectroscopy, and secondary ion mass spectrometry may be used. Still alternatively, a sample obtained by mechanically scraping off the surface of each region may be chemically analyzed for detection of elements. For example, phosphorus, calcium, antimicrobial components, and the like are detected from the surface of at least part of the first region 1 and the surface of at least part of the fourth region 4 where the coating film 20 is disposed. From the surface of the fifth region 5, elements constituting the base 10 are detected, and phosphorus, calcium, antimicrobial components, and the like are not detected, or are detected at a noise level or lower.

The surface of the coating film 20 located in the fourth region 4 may have a smaller surface roughness than the surface of the coating film 20 located in the first region 1. Accordingly, physical irritation to soft tissue can be reduced in the fourth region 4.

Note that the surface roughness (Sa) of the coating film 20 located in the fourth region 4 is only required to be set to, for example, 0.1 to 10 µm.

The surface of the coating film 20 located in the fourth region 4 may have a larger surface roughness than the outer surface of the base 10 in the fifth region 5. Note that the surface roughness (Sa) of the base 10 in the fifth region 5 is only required to be set to, for example, less than 1 µm .

The surface roughness of the outer surface of the base 10 in the fourth region 4 may be larger than the surface roughness of the outer surface of the base 10 in the third region 3 or the fifth region 5. Accordingly, in the fourth region 4, the adhesion between the coating film 20 and the base 10 can be improved, and thus the peeling of the coating film 20 can be reduced. Note that the surface roughness (Sa) of the base 10 in the fourth region 4 is only required to be set to 0.1 µm or more and less than 10 µm, and may be set to less than 2.0 µm.

### 2. Method for Manufacturing Artificial Joint Shell

In an embodiment, a method for manufacturing an artificial joint shell includes preparing a base having a cup shape, disposing a first protective material such that a first part of an outer surface of the base is exposed and a second part of the outer surface is protected (S101), a first surface roughening step of forming a first rough surface portion at the first part exposed from the first protective material (S102), removing the first protective material (S103), and a coating film forming step of forming a coating film 20 containing a calcium phosphate-based material and an antimicrobial material at the first rough surface portion and at least a part of the second part of the outer surface (S108).

Accordingly, as described above, the artificial joint shell can be obtained in which the coating film 20 is located across the first region 1 and the second region 2, and the surface of the coating film 20 located in the first region 1 has a larger surface roughness than the surface of the base 10 in the second region 2.

The base having a cup shape can be formed, for example, by a forming process such as casting, plastic forming, or sintering when metal is used as a material. After formation in the forming process, a removal process such as cutting, grinding, or electric spark machining may be performed for shape adjustment. The screw hole 31 or the counterbored hole 32 may be formed in the forming process, or may be formed in the removal process, not in the forming process.

In the first surface roughening step, the rough surface can be formed by at least one selected from the group consisting of thermal spraying, additive manufacturing, chemical etching, and blasting. The thermal spraying, the additive manufacturing, or the chemical etching can produce larger surface roughness than the blasting. As materials for the thermal spraying and the additive manufacturing, the materials that have been exemplified as the materials of the base 10 can be used. The above-described layered structure may be formed by the thermal spraying or the additive manufacturing. Examples of the chemical etching include alkali treatment. Examples of the blasting include sandblasting. Note that the rough surface may be formed before the coating film 20 is formed.

The coating film 20 can be formed by a thermal spraying such as flame spraying, high-speed flame spraying, and plasma spraying. In addition, physical vapor deposition or chemical vapor deposition such as sputtering, ion plating, ion beam deposition, and ion mixing can also be used. Also, wet coating method such as sol-gel method can be used.

A protective material may be used to form the rough surface or the coating film 20 only in a desired region. For example, a masking tape or a screen may be used as the protective material. Alternatively, a jig covering the base 10 may be used as the protective material. Examples of materials of these protective materials include a metal, a glass, a resin, and a composite thereof.

Note that the protective material may be or need not be in contact with the base 10. When the protective material is not in contact with the base 10, some quantity of a thermal spraying material, a coating film material, or the like may enter a region covered with the protective material from around an edge of the protective material and adheres to the region. Accordingly, the layered member 30 including the edge having a lower height than the inner portion thereof, the coating film 20 including the base portion 22 having a larger thickness than the end portion 21, and/or the like, may be formed as described above. As the protective material, for example, a masking tape having adhesiveness and capable of being attached to the base 10, or a jig covering the base 10 described below can be used.

In the case where a screen is disposed, the rough surface or the coating film 20 can be formed in a specific region by moving the position of the screen each time the rough surface or the coating film 20 is formed. In the case where a jig is used, the rough surface or the coating film 20 can be formed in a specific region by changing a region covered by the jig each time the rough surface or the coating film 20 is formed. In this case, the rough surface or the coating film 20 may also be selectively formed only in a desired region by, for example, adjusting the positional relationship between the screen and a discharge nozzle discharging a thermal spraying material or a coating material. In this case, a tip of the discharge nozzle is only required to be arranged, for example, in a straight line with the surface of the desired region without being separated by the screen. Note that the coating material means a material constituting the coating film. In the first surface roughening step, the surface of the first region 1 and the tip of the discharge nozzle is only required to be arranged in a straight line without being separated by the screen. In the coating film forming step, the surface of a region extending across the first region 1 and the second region 2 and the tip of the discharge nozzle are only required to be arranged in a straight line without being separated by the screen. In the case where the screw hole 31 and/or the counterbored hole 32 are/is provided in the base 10, the surface of a region across the first region 1 and the second region 2, the surface of the fourth region 4, and the tip of the discharge nozzle may be arranged in a straight line without being separated by the screen. In the case where the coating film 20 is formed simultaneously in the first region 1 and the second region 2, the tip of the discharge nozzle may be arranged, for example, in a straight line with the surface of the first region 1 without being separated by the screen in view of material diffusion. Without being limited to the above, the rough surface or the coating film 20 may be formed in a state where the base 10, the screen, and the discharge nozzle are fixed, or the rough surface or the coating film 20 may be formed while at least one selected from the group consisting of the base 10, the screen, and the discharge nozzle is moved. Also, the rough surface or the coating film 20 may be formed with the angle of the discharge nozzle fixed or changed.

Note that the rough surface or the coating film 20 can be formed only in a desired region without using a protective material. For example, the rough surface or the coating film 20 can be selectively formed only in a desired region by adjusting the shape, the angle, the position or the like of the discharge nozzle discharging a thermal spraying material or coating material. For example, the thermal spraying material or the coating material may be discharged in a state where the discharge nozzle is located above the surface of the desired region. In the first surface roughening step, the thermal spraying material may be discharged in a state where the discharge nozzle is located above the surface of the first region 1. In the coating film forming step, the coating material may be discharged in a state where the discharge nozzle is located above the surface of the region across the first region 1 and the second region 2. In the case where the screw hole 31 and/or the counterbored hole 32 are/is provided in the base 10, the coating material may be discharged in a state where the discharge nozzle is located above the surface of the region across the first region 1 and the second region 2, and the surface of the fourth region 4. In that case, the rough surface or the coating film 20 may be formed while the base 10 is fixed and the position and the angle of the discharge nozzle are changed, or the rough surface or the coating film 20 may be formed while the discharge nozzle is fixed and the position and the angle of the base 10 are changed. The discharge nozzle may be moved at a constant speed or at a variable speed. A discharge direction of the thermal spraying material or the coating material may form an angle of 90° or an angle of less than 90° with respect to a vector extending from the tip of the discharge nozzle toward the surface of the base 10 or the rough surface located at the shortest distance from the tip of the discharge nozzle.

For example, in the first surface roughening step, the first protective material may be disposed at the base 10 such that the second region 2 is protected while the first region 1 is exposed, and the rough surface may be formed in the exposed first region 1. In the case where the screw hole 31 and/or the counterbored hole 32 are/is provided in the base 10, the first protective material may be disposed such that the second region 2, the fourth region 4, and the fifth region 5 are protected while the first region 1 is exposed, and the rough surface may be formed in the exposed first region 1.

In addition, in the present disclosure, the manufacturing method may further include a step of removing the first protective material after the first surface roughening step and before the coating film forming step.

After the first protective material is removed, a step of cutting the edge of the rough surface, for example, the edge of the layered member 30 may be performed. Accordingly, the concentration of stress at the edge of the layered member 30 can be avoided, and irritation to living tissue can be reduced.

A first masking tape may be used as the first protective material. In that case, the manufacturing method according to the present disclosure may further include a step of attaching the first masking tape to the second region 2 while exposing the first region 1 before the first surface roughening step. In the case where the screw hole 31 and/or the counterbored hole 32 are/is provided in the base 10, the manufacturing method may further include a step of attaching the first masking tape to the second region 2, the fourth region 4, and the fifth region 5 while exposing the first region 1.

Further, in the present disclosure, the manufacturing method may further include, after the step of removing the first protective material and before the coating film forming step, a step of disposing a second protective material such that a part of the second part of the outer surface is exposed and another part of the second part of the outer surface is protected (S104), and a second surface roughening step (S105) of forming a second rough surface portion at at least a part of the second part of the outer surface, and the second rough surface portion may be formed at the part exposed from the second protective material. In addition, the manufacturing method may further include a step of removing the second protective material (S106) after the second surface roughening step.

In the second surface roughening step, the rough surface may be formed by at least one selected from the group consisting of chemical etching and blasting. The second surface roughening step may be performed such that the surface of the second rough surface portion formed in the second surface roughening step has a smaller surface roughness than a first rough surface portion in the first region 1. Note that in the case of forming the second rough surface portion, the rough surface in the first region 1 can also be referred to as the first rough surface portion.

A second masking tape may be used as the second protective material. In that case, the manufacturing method according to the present disclosure may further include, before the second surface roughening step, a step of attaching the second masking tape to the third region 3 while exposing the first region 1 and the second region 2. In the case where the screw hole 31 and/or the counterbored hole 32 are/is provided in the base 10, the manufacturing method may further include a step of attaching a masking tape to the third region 3 and the fifth region 5 while exposing the first region 1 and the fourth region 4.

In the present disclosure, the manufacturing method may further include, after the step of removing the second protective material and before the coating film forming step, a step of disposing a third protective material such that the first rough surface portion and a part of the second rough surface portion are exposed and another part of the second rough surface portion and the outer surface are protected (S107). Accordingly, in the coating film forming step, the coating film can be formed at the first rough surface portion and the part of the second rough surface portion exposed from the third protective material. In the case where the screw hole 31 and/or the counterbored hole 32 are/is provided in the base 10, the manufacturing method may further include a step of disposing the third protective material such that a part of the second rough surface portion and the fourth region 4 are exposed and another part of the second rough surface portion, the fifth region 5, and the outer surface are protected (S107). Accordingly, in the coating film forming step, the coating film can be formed at the first rough surface portion, the part of the second rough surface portion, and the fourth region 4 exposed from the third protective material.

A third masking tape may be used as the third protective material. In that case, the manufacturing method according to the present disclosure may further include, before the coating film forming step, a step of attaching the third masking tape such that the first region 1 and a part of the second region 2 are exposed, the third masking tape being attached to another part of the second region 2. In the case where the screw hole 31 and/or the counterbored hole 32 are/is provided in the base 10, the manufacturing method may further include a step of attaching a masking tape such that the first region 1, a part of the second region 2, and the fourth region are exposed, the masking tape being attached to another part of the second region 2 and the fifth region 5.

In the present disclosure, the manufacturing method may include a hole forming step of forming a hole in the base in the step of preparing the base.

Note that in the second surface roughening step, the first region 1 may be or need not be covered with the protective material. The first region 1 may be protected and only the second region 2 may be subjected to at least one selected from the group consisting of chemical etching and blasting. In the case where the screw hole 31 and/or the counterbored hole 32 are/is provided in the base 10, the second region 2 and the fourth region may be subjected to at least one selected from the group consisting of chemical etching and blasting. Alternatively, the second protective material may be disposed so as to expose the first region 1, and a rough surface of the exposed first region 1 and the second region 2 may be subjected to at least one selected from the group consisting of chemical etching and blasting. In the case where the screw hole 31 and/or the counterbored hole 32 are/is provided in the base 10, a rough surface of the first region 1, the second region 2, and the fourth region may be subjected to at least one selected from the group consisting of chemical etching and blasting. Accordingly, an unnecessary thermal spraying material or the like remaining on the rough surface of the first region 1 can be removed and a rough surface can be formed in the second region 2. Further, in the case where the screw hole 31 and/or the counterbored hole 32 are/is provided in the base 10, a rough surface can be formed in the second region 2 and the fourth region.

Note that the second rough surface portion can be formed only in a desired region without using a protective material. For example, the second rough surface portion can be selectively formed only in a desired region by adjusting the shape, the angle, the position or the like of a discharge nozzle discharging a chemical etching material or blasting material. Note that the chemical etching material means a material that is discharged toward the base 10 in the case of performing processing by chemical etching. Similarly, the blasting material means a material that is discharged toward the base 10 in the case of performing processing by blasting. For example, the chemical etching material or the blasting material may be discharged in a state where the discharge nozzle is located above the surface of the desired region. In the case where the second rough surface portion is formed only in the second region 2, the chemical etching material or the blasting material may be discharged in a state where the discharge nozzle is located above the surface of the second region 2. In the case where the second rough surface portion is formed in the first region 1 and the second region 2, the chemical etching material or the blasting material may be discharged in a state where the discharge nozzle is located above the surfaces of the first region 1 and the second region 2. In the case where the screw hole 31 and/or the counterbored hole 32 are/is provided in the base 10 and where the second rough surface portion is formed in the first region 1, the second region 2, and the fourth region 4, the chemical etching material or the blasting material may be discharged in a state where the discharge nozzle is located above the surfaces of the first region 1, the second region 2, and the fourth region 4. In that case, the second rough surface portion may be formed while the base 10 is fixed and the position and the angle of the discharge nozzle are changed, or the second rough surface portion may be formed while the discharge nozzle is fixed and the position and the angle of the base 10 are changed. The discharge nozzle may be moved at a constant speed or at a variable speed. A discharge direction of the chemical etching material or the blasting material may form an angle of 90° or an angle of less than 90° with respect to a vector extending from the tip of the discharge nozzle toward the surface of the base 10 or the rough surface located at the shortest distance from the tip of the discharge nozzle.

For example, thermal spraying may be used in the first surface roughening step and the coating film forming step, and blasting may be used in the second surface roughening step. In that case, a material having a higher thermal resistance than the second protective material can be used as the first protective material. Also, a material having a higher thermal resistance than the third protective material can be used as the first protective material. Also, a material having a higher thermal resistance than the second protective material can be used as the third protective material. In order to satisfy the above-described relationship, for example, a material that is not dissolved or thermally decomposed for one minute at a thermal spraying condition of 8000°C may be used as the first protective material, a material that is not dissolved or thermally decomposed for one minute at a thermal spraying condition of 3000°C may be used as the third protective material, and a material that is not dissolved or thermally decomposed at a room temperature may be used as the second protective material. As specific examples of such materials, a composite material of a glass and a resin may be used as the first protective material, a composite material of a glass and a resin may be used as the third protective material, and a resin may be used as the second protective material.

In the case where a jig covering the base 10 is used as a protective material, the shape of the jig is not particularly limited, but may be, for example, tubular. The cross-section of a jig having a tubular shape may be polygonal, or may be circular.

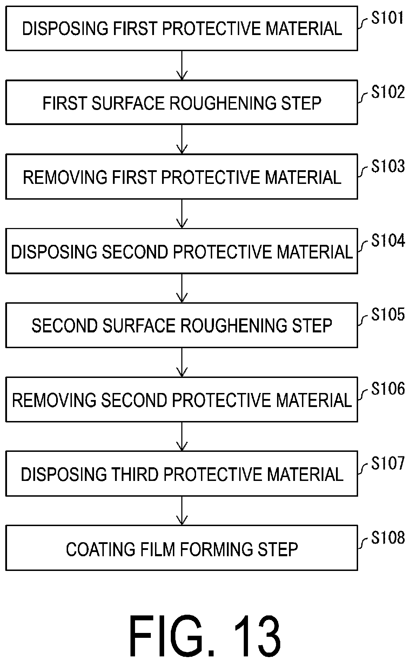

In summary, for example, processing steps can be performed in the order illustrated in FIG. 13. FIG. 13 is a flowchart illustrating a method for manufacturing an artificial joint shell according to an embodiment. First, the first protective material is disposed, the first surface roughening step is performed, and then the first protective material is removed. Subsequently, the second protective material is disposed, the second surface roughening step is performed, and then the second protective material is removed. Then, the third protective material is disposed and then the coating film forming step is performed.

Further, in the present disclosure, the manufacturing method may include or need not include a cleaning step between respective steps. For example, in the present disclosure, the manufacturing method may include a step of cleaning the base 10, or the base 10 and the layered member 30 disposed on the first region 1 after the first surface roughening step. Also, the manufacturing method may further include a step of cleaning the base 10, or the base 10 and the layered member 30 disposed on the first region 1 after the second surface roughening step. A cleaning method is not particularly limited, and may be, for example, a method of immersing in a liquid such as water or an organic solvent such as alcohol, or may be a method of showering using the liquid. Alternatively, a method of blowing a gas such as air, nitrogen, or argon may be used. Accordingly, an unnecessary thermal spraying material and the like generated during the first surface roughening step and/or cutting chips and the like generated during the second surface roughening step can be removed.

### 3. Use of Artificial Joint Shell

The artificial joint shell 130 illustrated in FIG. 1 has a shape mainly assumed to be the shape of an artificial hip joint shell, but an artificial joint to which the artificial joint shell according to the present disclosure is applied is not limited to an artificial hip joint. Examples of the artificial joint include an artificial hip j oint, an artificial shoulder j oint, and an artificial elbow joint.

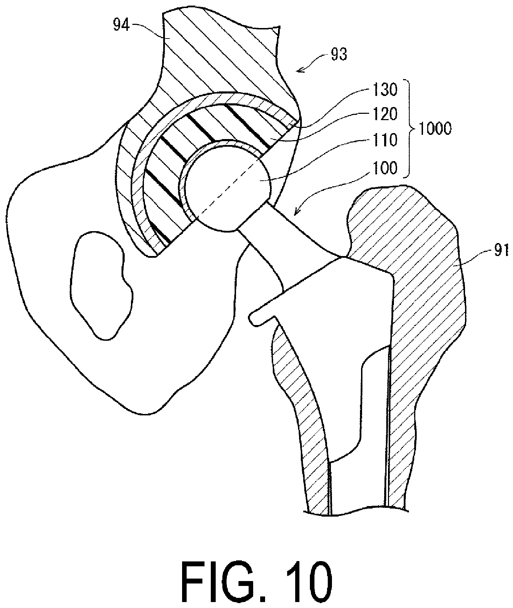

In the following, an example in which the artificial joint shell 130 is used as a part of an artificial hip joint 1000 will be described with reference to FIG. 10. The artificial hip joint 1000 may include an artificial joint stem 100, a bone head 110, a liner 120, in addition to the artificial joint shell 130. The artificial joint stem 100 and the bone head 110 may be made of the same material as, or a different material from the material of the base 10 of the artificial joint shell 130. The artificial joint stem 100 is embedded in a thighbone 91. The bone head 110 is disposed at an exposed portion of the artificial joint stem 100. The artificial joint shell 130 is secured to an acetabulum 94 of the coxal bone 93, and the bone head 110 is fitted into a recess of the artificial joint shell 130 and is slid to function as a hip joint. To be more precise, the bone head 110 is fitted into a recess of the liner 120 fitted in the opening portion 12 of the artificial joint shell 130 and is slid to function as a hip joint.

The invention according to the present disclosure has been described above based on the drawings and examples. However, the invention according to the present disclosure is not limited to each embodiment described above. That is, the invention according to the present disclosure can be variously modified within the scope indicated in the present disclosure, and an embodiment to be obtained by appropriately combining technical means disclosed in different embodiments is also included in the technical scope of the invention according to the present disclosure. In other words, note that a person skilled in the art can easily make various variations or modifications based on the present disclosure. Also note that these variations or modifications are included within the scope of the present disclosure.

### REFERENCE SIGNS

1 First region
2 Second region
3 Third region
4 Fourth region
5 Fifth region
10 Base
20 Coating film
21 End portion of coating film
22 Base portion of coating film
30 Layered member
100 Artificial joint stem
110 Bone head
120 Liner
130 Artificial joint shell
1000 Artificial hip joint

## Claims

1. An artificial joint shell comprising:
a base having a cup shape, the base comprising an outer surface, the outer surface comprising a first region and a second region adjacent to the first region; and
a coating film containing a calcium phosphate-based material and an antimicrobial material, the coating film being disposed across the first region and the second region of the outer surface of the base, wherein
a surface of the coating film in the first region has a larger surface roughness than a surface of the coating film in the second region.

2. The artificial joint shell according to claim 1, wherein
the second region is disposed between the first region and an opening portion of the base.

3. The artificial joint shell according to claim 2, further comprising:
a layered member, the layered member being disposed in the first region.

4. The artificial joint shell according to claim 3, wherein
the layered member is made of metal.

5. The artificial joint shell according to claim 3 or 4, wherein
the coating film disposed in the first region is disposed on the layered member.

6. The artificial joint shell according to any one of claims 3 to 5, wherein
the layered member comprises an edge having a lower height than an inner portion of the layered member.

7. The artificial joint shell according to any one of claims 2 to 6, wherein
the coating film located in the second region comprises an end portion and a base portion located closer to the first region than the end portion is to the first region, and
a thickness of the base portion is larger than a thickness of the end portion.

8. The artificial joint shell according to any one of claims 2 to 6, wherein
the outer surface of the base further comprises a third region located adjacent to the second region and between the second region and the opening portion, and
the outer surface of the base located in the third region is exposed from the coating film.

9. The artificial joint shell according to claim 8, wherein
the surface of the coating film in the second region has a larger surface roughness than the outer surface in the third region.

10. The artificial joint shell according to any one of claims 2 to 9, wherein
the outer surface in the first region has a larger surface roughness than the outer surface in the second region.

11. The artificial joint shell according to claim 8 or 9, wherein
the outer surface in the second region has a larger surface roughness than the outer surface in the third region.

12. The artificial joint shell according to any one of claims 2 to 11, wherein
the base comprises at least one through hole penetrating the outer surface and an inner surface of the base.

13. The artificial joint shell according to claim 12, wherein
the outer surface of the base further comprises a fourth region located adjacent to the first region and between the first region and the at least one through hole, and
the outer surface located in the fourth region has a smaller surface roughness than the surface of the coating film in the first region.

14. The artificial joint shell according to claim 13, wherein
at least one of the at least one through hole is a screw hole disposed at a position further from the opening portion than other positions, and
at least one of the at least one through hole other than the screw hole is a counterbored hole comprising a first penetrating portion and a second penetrating portion, the second penetrating portion being connected to the first penetrating portion and the outer surface and having a narrower width than the first penetrating portion.

15. The artificial joint shell according to claim 14, wherein
the coating film is disposed at at least a part of the outer surface in the fourth region.

16. The artificial joint shell according to claim 14 or 15, wherein
the outer surface of the base further comprises a fifth region located adjacent to the fourth region and between the fourth region and the screw hole, and
the outer surface in the fifth region is fully exposed from the coating film.

17. The artificial joint shell according to claim 14, wherein
the fourth region is adjacent to the counterbored hole.

18. A method for manufacturing an artificial joint shell, the method comprising:
preparing a base having a cup shape;
disposing a first protective material such that a first part of an outer surface of the base is exposed and a second part of the outer surface is protected;
first surface roughening of forming a first rough surface portion at the first part exposed from the first protective material;
removing the first protective material; and
coating film forming of forming a coating film at the first rough surface portion and at least a part of the second part of the outer surface, the coating film containing a calcium phosphate-based material and an antimicrobial material.

19. The method for manufacturing an artificial joint shell according to claim 18, the method further comprising:
second surface roughening of forming a second rough surface portion at at least a part of the second part of the outer surface, after the removing of the first protective material and before the coating film forming.

20. The method for manufacturing an artificial joint shell according to claim 19, the method further comprising:
after the removing of the first protective material and before the second surface roughening, disposing a second protective material such that a part of the second part of the outer surface is exposed and another part of the second part of the outer surface is protected, wherein
the second surface roughening comprises forming the second rough surface portion at the part exposed from the second protective material.

21. The method for manufacturing an artificial joint shell according to claim 20, the method further comprising:
removing the second protective material after the second surface roughening and before the coating film forming.

22. The method for manufacturing an artificial joint shell according to claim 21, the method further comprising:
after the removing of the second protective material and before the coating film forming, disposing a third protective material such that the first rough surface portion and a part of the second rough surface portion are exposed and another part of the second rough surface portion and the outer surface are protected, wherein
the coating film forming comprises forming the coating film at the first rough surface portion and the part of the second rough surface portion exposed from the third protective material.

23. The method for manufacturing an artificial joint shell according to any one of claims 20 to 22, wherein
the disposing of the second protective material comprises disposing the second protective material such that the first rough surface portion is exposed, and
the second surface roughening comprises processing the first rough surface portion exposed from the second protective material by blasting.

24. The method for manufacturing an artificial joint shell according to claims 20 to 23, wherein
a material having a higher thermal resistance than the second protective material is used as the first protective material.

25. The method for manufacturing an artificial joint shell according to any one of claims 22 to 24, the method further comprising:
after the removing of the second protective material and before the coating film forming, disposing the third protective material such that the first rough surface portion and a part of the second rough surface portion are exposed and another part of the second rough surface portion and the outer surface are protected, wherein
a material having a higher thermal resistance than the third protective material is used as the first protective material.

26. The method for manufacturing an artificial joint shell according to any one of claims 22 to 25, the method further comprising:
after the removing of the second protective material and before the coating film forming, disposing the third protective material such that the first rough surface portion and a part of the second rough surface portion are exposed and another part of the second rough surface portion and the outer surface are protected, wherein
a material having a higher thermal resistance than the second protective material is used as the third protective material.

27. The method for manufacturing an artificial joint shell according to any one of claims 20 to 26, wherein
the disposing of the first protective material further comprises attaching a first masking tape to the second part of the outer surface such that the first part of the outer surface is exposed, the first masking tape being used as the first protective material.

28. The method for manufacturing an artificial joint shell according to any one of claims 20 to 27, wherein
the disposing of the second protective material further comprises attaching a second masking tape to the other part of the second part of the outer surface such that the part of the second part of the outer surface is exposed, the second masking tape being used as the second protective material.

29. The method for manufacturing an artificial joint shell according to any one of claims 22 to 28, the method further comprising:
after the removing of the second protective material and before the coating film forming, disposing the third protective material such that the first rough surface portion and a part of the second rough surface portion are exposed and another part of the second rough surface portion and the outer surface are protected, wherein
the disposing of the third protective material further comprises attaching a third masking tape to the other part of the second rough surface portion and the outer surface such that the part of the second rough surface portion is exposed, the third masking tape being used as the third protective material.

30. The method for manufacturing an artificial joint shell according to any one of claims 20 to 29, wherein
the preparing of the base comprises hole forming of forming a hole in the base.

31. The method for manufacturing an artificial joint shell according to claim 30, the method further comprising:
after the removing of the first protective material and before the coating film forming,
disposing the second protective material such that a part of the second part of the outer surface is exposed and another part of the second part of the outer surface is protected,
the second surface roughening of forming the second rough surface portion at the part exposed from the second protective material, and
disposing a third protective material such that the first rough surface portion and a part of the second rough surface portion are exposed and another part of the second rough surface portion and the outer surface are protected, wherein
the first surface roughening comprises protecting an edge of the hole by the first protective material,
the second surface roughening comprises exposing the edge of the hole from the second protective material, and
the coating film forming comprises exposing the edge of the hole from the third protective material.

32. The method for manufacturing an artificial joint shell according to any one of claims 19 to 31, wherein
the first surface roughening is performed by at least one selected from the group consisting of thermal spraying and blasting.

33. The method for manufacturing an artificial joint shell according to any one of claims 19 to 32, wherein
the second surface roughening is performed by blasting.
